# EUROPÄISCHE PATENTSCHRIFT

(11) **EP 0 912 765 B1**
(45) Veröffentlichungstag und Bekanntmachung des Hinweises auf die Patenterteilung: **31.07.2002**
(21) Anmeldenummer: 97930488.8
(22) Anmeldetag: 02.07.1997
(51) Int. Cl.: C12Q 1/68, G01N 33/58

(54) **BESTIMMUNG VON ANALYTEN UNTER VERWENDUNG ZWEIER MARKIERUNGEN**
DETERMINATION OF ANALYTES BY MEANS OF TWO MARKERS
DETECTION D'ANALYTES AU MOYEN DE DEUX MARQUEURS

(30) Priorität: 06.07.1996 DE 19627290
(43) Veröffentlichungstag der Anmeldung: 06.05.1999
(73) Patentinhaber: Roche Diagnostics GmbH, 68298 Mannheim (DE)
(72) Erfinder: GIESEN, Ursula, D-82362 Weilheim (DE); WENZIG, Peter, D-81369 München (DE); ZIEGLER, Günter, D-82398 Polling (DE); WEINDEL, Kurt, D-82407 Wielenbach (DE)
(86) Internationale Anmeldenummer: EP9703480
(87) Internationale Veröffentlichungsnummer: WO9801578

(56) Entgegenhaltungen:
- EP-A- 0 617 288
- EP-A- 0 709 466
- WO-A-92/12255
- WO-A-93/12256
- WO-A-94/01774
- DE-A- 3 022 426
- NELSON N C ET AL: "SIMULTANEOUS DETECTION OF MULTIPLE NUCLEIC ACID TARGETS IN A HOMOGENEOUS FORMAT" BIOCHEMISTRY, Bd. 35, Nr. 25, 25.Juni 1996, Seiten 8429-8438, XP000606273
- GLASER V: "CHEMILUMINESCENCE SHINES IN INFECTIOUS DISEASE DIAGNOSIS AND IDENTITY TESTING" GENETIC ENGINEERING NEWS, Bd. 16, 1.Juli 1996, Seite 3, 17 XP000606011

## Beschreibung

Gegenstand der vorliegenden Erfindung ist ein Verfahren zur Bestimmung eines Analyten in einer Probe, ein Reagenzkit zur Ausführung dieses Verfahrens und die Verwendung zweier unterschiedlich markierter Sonden zum quantitativen Nachweis von Analyten in einer Probe.

Der Nachweis von Analyten in Proben hat insbesondere im Gesundheitswesen eine erhebliche Bedeutung erlangt. Viele Analyten, z. B. in Körperflüssigkeiten, können als Indiz für das Vorliegen einer Erkrankung oder Infektion verwendet werden. Viele der Analyten sind jedoch selbst nicht direkt nachweisbar oder sind in sehr geringen Mengen neben großen Mengen ganz ähnlicher Komponenten in der Probe enthalten, so daß ein direkter Nachweis in vielen Fällen praktisch unmöglich ist. Aus diesem Grund wird immer öfter versucht, die zu bestimmenden Analyten mit Hilfe nachweisbar markierter Sonden spezifisch nachweisbar zu machen. Diese Sonden binden im Idealfall nur den Analyten und markieren ihn über diese Bindung. Mittlerweile stehen eine Vielzahl von Markierungsgruppen zur Verfügung. Dazu gehören beispielsweise Metalle, farbige Verbindungen, fluoreszierende Verbindungen, aber auch Enzyme, Elektrolumineszenzgruppen und chemisch aktivierbare Gruppen.

In WO 92/14139 ist beispielsweise ein Gerät zur Durchführung eines Bindetests für einen zu bestimmenden Analyten beschrieben, welches auf Elektrochemilumineszenz beruht. Hierbei wird der Analyt über seine Bindung an eine mit Hilfe eines Metallkomplexes markierte Sonde und Anregung durch Anlegen einer Spannung sowie Detektion des erzeugten Lichtsignales bestimmt.

Ebenfalls als Markierungsgruppe wurden kalziumaktivierbare Photoproteine vorgeschlagen, z. B. in EP 764 468. Der Mechanismus der Lichterzeugung beruht bei diesen Proteinen darauf daß eine Lösung, welche den über einen mit Hilfe einer aequorinmarkierten Sonde markierten Analyten enthält, eine kalziumsalzhaltige Lösung zugegeben wird. Hierdurch wird die Erzeugung eines elektromagnetischen Signals aus dem Aequorin getriggert.

Nukleinsäuren sind aufgrund ihrer in der Basensequenz gespeicherten Information besonders wertvolle Hilfsmittel in der Diagnostik. Spezielle Nukleinsäuresequenzen liegen jedoch gerade deshalb in sehr großem Unterschuß gegenüber ganz ähnlichen Sequenzen vor. Es hat sich daher als vorteilhaft erwiesen, die nachzuweisenden Nukleinsäuren vor ihrem Nachweis spezifisch zu amplifizieren, d. h. eine Vielzahl von Kopien einer bestimmten Sequenz herzustellen. Ein solches Verfahren stellt die Polymerasekettenreaktion (PCR) dar (US-A-4,683,202). Gerade bei dieser Amplifikationsreaktion hat es sich jedoch erwiesen, daß ein quantitativer Nachweis von Nukleinsäuren nicht oder nur unter sehr günstigen Bedingungen möglich ist, da die Amplifikationseffizienz sehr stark von unterschiedlichsten Faktoren beeinflußt wird. Es wurde daher vorgeschlagen, den analythaltigen Proben vor der Amplifikation einen Standardanalyten in bekannter Menge zuzusetzen, der sich in einer detektierbaren Eigenschaft von dem zu bestimmenden Analyten unterscheidet, sich jedoch in Bezug auf die Amplifikationseffizienz ähnlich verhält wie der Analyt. Eine solche Polymerasekettenreaktion unter Verwendung eines internen Standards ist beispielsweise beschrieben in US-A-5,213,961 oder US-A-5,219,727. Ähnliche Verfahren sind beschrieben in WO 92/01812, WO 94/04706, EP-A-0 *525* 882, WO 95/02067 und WO 94/09156. In letztgenannter Patentanmeldung wird ein Verfahren beschrieben, bei dem in der PCR immobilisierbare Primer eingesetzt werden und die Menge an Amplifikaten über Sonden nachgewiesen wird.

In WO 93/10257 ist ein Verfahren beschrieben, bei dem zunächst eine PCR durchgeführt und die Reaktionsmischung anschließend mit zwei unterschiedlich markierten Sonden inkubiert wird.

In WO 89/10552 ist ein Apparat zur simultanen Messung zweier Proben mittels verschiedener ECL-anregbarer Label beschrieben. Es werden zwei Messzellen und zwei Detektoren verwendet, die unterschiedliche Wellenlängen generieren und detektieren. Verfahren, bei denen die Anregung räumlich und zeitlich getrennt funktioniert, um eine ausreichende Dynamik und Sensitivität zu erzielen, haben jedoch den Nachteil, daß der Probendurchsatz gering und die benötigten Probenvolumina groß sind.

In DE-C-3022426 ist ein Immunoassay beschrieben, bei dem ein Chemilumineszenzmarker durch Anlegen einer Spannung und dadurch erzeugtes Oxidans zur Emission angeregt wird.

In EP-0 478-626 wird eine Detektionsgruppe beschrieben, die durch unterschiedliche Substanzen so modifiziert wird, daß die daraus resultierenden Detektionsgruppen ein unterschiedliches kinetisches Verhalten oder ein unterschiedliches Spektrum aufweisen. Die Anregung erfolgt gleichzeitig durch denselben Trigger (oxidativ). Die Signale (spektral oder kinetisch) überlappen sich jedoch in der Praxis sehr stark, so daß eine verringerte Dynamik und eine erniedrigte Sensitivität resultiert.

In EP-0-199 804, US-5,238,808 und US-5,310,687 ist ebenfalls ein Mehrfach-Markierungskonzept beschrieben, bei dem die unterschiedlichen Label optisch aufgrund ihrer unterschiedlichen spektralen Charakteristika detektiert werden.

In WO 93/01308 ist ein Verfahren zum Nachweis eines Analyten mittels Acridiniumester-markierter Antikörper und Erzeugung eines Chemilumineszenzsignals durch Oxidation bei basischem pH-Wert beschrieben.

Ein Ziel der vorliegenden Erfindung war es daher, den Stand der Technik teilweise oder vollständig zu verbessern und insbesondere Verfahren bereitzustellen, bei denen der Nachteil der bei gleichzeitiger Fluoreszenzanregung nicht oder schlecht zu trennenden Signale bei gleichzeitig erhöhtem Durchsatz gegenüber getrennt nacheinander geführten Nachweisreaktionen vermieden wird.

Gegenstand der Erfindung ist daher ein Verfahren zur Bestimmung eines Analyten in einer Probe durch Inkubation der Probe mit mindestens zwei Sonden, von denen mindestens eine für den zu bestimmenden Analyten spezifisch ist und wobei die mindestens zwei Sonden unterschiedliche Markierungsgruppen tragen, Erzeugung jeweils eines unterschiedlichen elektromagnetischen Signals für jede unterschiedliche Markierungsgruppe und Auswertung der erzeugten Signale als Zeichen für die Anwesenheit oder Menge des Analyten. Ebenfalls Gegenstand der Erfindung ist ein Reagenzkit zur Bestimmung eines Analyten und die Verwendung zweier Sonden, die ein unterschiedliches elektromagnetisches Signal liefern können, zum quantitativen Nachweis von Analyten in einer Probe.

In Figur 1 ist der schematische Aufbau eines Gerätes zur Durchführung des erfindungsgemäßen Verfahrens gezeigt.

In Figur 2 ist der Signalverlauf für eine erfindungsgemäße Bestimmung bei unterschiedlichen Analytkonzentrationen gezeigt.

In Figur 3 ist ein Vergleich zwischen zwei erfindungsgemäßen Verfahren gezeigt, bei denen die Detektionsreaktionen seitlich vertauscht wurden.

In Figur 4 ist die Struktur eines für die Anknüpfung eines Labels verwendeten Linkermoleküls gezeigt.

In Figur 5 und 6 ist gezeigt, daß die Bestimmungen völlig unabhängig voneinander ablaufen.

In Figur 7 und 8 ist das Ergebnis eines Kompetitionsversuches zweier unterschiedlich markierter Sonden um dasselbe Target gezeigt.

Unter einem Analyt im Sinne der Erfindung wird alles Material verstanden, welches Gegenstand des Bestimmungsverfahrens sein soll. Es handelt sich bevorzugt um Inhaltsstoffe von Proben, wie sie üblicherweise in der medizinischen Diagnostik anfallen, d. h. insbesondere Inhaltsstoffe von Körperflüssigkeiten. Hierzu gehören insbesondere Antigene, Antikörper, Zellen oder Nukleinsäuren. Bei den Nukleinsäuren kann es sich um Nukleinsäuren handeln, die für eine Infektion, z. B. viralen oder baktierellen Ursprungs, spezifisch sind, z. B. virale oder bakterielle Nukleinsäuren oder körpereigene Nukleinsäuren, bei denen untersucht werden soll, ob sie sich gegenüber dem normalen Zustand verändert haben, z. B. durch Mutation, Deletion oder Insertion in einer oder mehreren Stellen. Virale Nukleinsäuren sind beispielsweise Ribonukleinsäuren von RNA-Viren, z. B. HCV, HIV oder HGV oder genomische DNA oder rRNA von Bakterien, z. B. Chlamydien, Neisserien oder Salmonellen.

Unter einer Probe im Sinne der Erfindung werden insbesondere flüssige Proben verstanden, in denen der zu bestimmende Analyt gelöst oder suspendiert ist. Bevorzugt ist die Probe eine Körperflüssigkeit, z. B. Blut, Urin oder Sputum oder eine davon abgeleitete Flüssigkeit, z. B.

Serum, Plasma, Buffy-Coat oder eine Flüssigkeit, die unter Durchführung eines oder mehrerer Reaktionsschritte davon abgeleitet wurde. Diese Reaktionsschritte finden bevorzugt unter Zugabe von Reagenzien statt und können eine Anreicherung, Modifizierung oder Vermehrung des in der ursprünglichen Probe vorhandenen Analyten oder die Abreicherung störender Bestandteile der ursprünglichen Probe bewirkt haben. Ein besonders bevorzugtes Probenmaterial sind Reaktionsmischungen, wie sie üblicherweise nach Durchführung einer Probenvorbereitung mit anschließender PCR vorliegen. Unter einer Sonde im Sinne der Erfindung wird eine Komponente eines Nachweissystemes für einen zu bestimmenden Analyten oder/und einen Standardanalyten verstanden. Solche Komponenten sind beispielsweise solche, die den Analyten oder den Standardanalyten über biologische Wechselwirkungen, z B. immunologische Wechselwirkungen oder Wechselwirkungen durch Basenpaarungen, zwischen fiir eine Hybridisierung ausreichend komplementären Sequenzen von Nukleobasen, erkennen. Bei der Sonde handelt es sich daher bevorzugt um einen Antikörper, ein Antigen, ein Hapten oder eine Nukleinsäure oder ein Nukleinsäureanaloges, welches sich z. B. dadurch von natürlichen Nukleinsäuren unterscheidet, daß das Zuckerphosphatgrundgerüst durch ein Peptidgrundgerüst ersetzt ist (z. B. gemäß WO 92/20702).

Das erfindungsgemäße Verfahren benutzt mindestens zwei Sonden, von denen mindestens eine für den zu bestimmenden Analyten spezifisch ist. Als spezifisch wird die Sonde verstanden, wenn sie unter den Testbedingungen weniger als 5 % Kreuzreaktivität mit anderen, z. B. nicht zu bestimmenden, Probenbestandteilen oder in der Probe zu erwartenden Bestandteilen aufweist, d. h. solche andere Bestandteile zu weniger als 5 % verglichen mit der Bindung des gewünschten Bestandteiles, z. B. des Analyten, bindet.

Bevorzugt ist mindestens eine weitere Sonde nicht für den zu bestimmenden Analyten spezifisch. Bevorzugt ist diese Sonde spezifisch für einen weiteren Inhaltsstoff der Probe, insbesondere einen Bestandteil, der sich in seiner Struktur nur geringfügig, jedoch in definiertem Maß von dem zu bestimmenden Analyten unterscheidet. Bevorzugt ist dieser weitere Bestandteil der Probe ein Standardanalyt.

Unter einem Standardanalyten im Sinne der Erfindung ist ein Material zu verstehen, welches in der Probe in einer definierten Menge, bevorzugt einer bekannten Menge, enthalten ist oder ihr zugesetzt wurde. Der Standardanalyt unterscheidet sich auf definierte Weise von dem Analyten, z. B. in seiner Bindefähigkeit zu der analytspezifischen Sonde, die er unter den gewählten Testbedingungen bevorzugt im wesentlichen nicht bindet. Dieser Standardanalyt kann bereits ursprünglich in der Probe enthalten sein, bevorzugt wird er der Probe jedoch vor Inkubation der Probe mit den Sonden zugegeben. Für den Fall einer Bestimmung einer spezifischen Nukleinsäure in einer Probe ist es bevorzugt, daß der Standardanalyt ebenfalls eine Nukleinsäure ist, die sich von der Analytnukleinsäure entweder in ihrer Nukleotidsequenz oder ihrer Länge unterscheidet. Besonders bevorzugt wird zur Herstellung einer Standardnukleinsäure nach rekombinanten Technologien ein Teilstück einer Nukleinsäure mit der Analytsequenz entfernt und hierfür ein Stück einer anderen, bevorzugt nicht im Analyten enthaltenen, Sequenz eingefügt. Für diesen Fall ist es nicht erforderlich, daß sich die Analytnukleinsäure in ihrer Länge in größerem Umfang von der Standardnukleinsäure unterscheidet. Bevorzugt wird sowohl ein Teil der Analytnukleinsäure als auch ein Teil der Standardnukleinsäure einem Amplifikationsverfahren, bevorzugt einem Verfahren zur Vermehrung dieser Sequenzen, z. B. mit Hilfe der Polymerasekettenreaktion nach US-A-4,683,202, unterzogen. Hierfür werden bevorzugt Primer eingesetzt, welche sowohl zur Amplifikation der Analytnukleinsäure als auch der Standardnukleinsäure geeignet sind. Dieses Verfahren wird im allgemeinen als kompetitive PCR bezeichnet und ist beispielsweise in US-A-5,213,961 beschrieben.

Für den oben genannten Fall einer Standardnukleinsäure wird die Nukleotidsequenz der ersten Sonde (Analytsonde) so gewählt, daß sie mit dem Analyten oder dem Arnplifikationsprodukt der Analytteilsequenz in einem Bereich hybridisieren kann, der in der Standardnukleinsäure nicht vorhanden ist, während die zweite Sonde (Standardsonde) in ihrer Sequenz so gewählt wird, daß sie mit dem Verlängerungsprodukt der Primer nur dann hybridisieren kann, wenn das Verlängerungsprodukt durch Primerelongation unter Verwendung der Standardnukleinsäure als Matrize gebildet wurde. Bevorzugt handelt es sich bei den Sonden um Oligonukleotide oder Peptidnukleinsäuren (PNA, WO 92/20702). Wenn die erste Sonde analytspezifisch ist und den Standardanalyten im wesentlichen nicht bindet, kann die zweite Sonde entweder so gewählt werden, daß sie für den Standardanalyten spezifisch ist, oder daß sich sowohl den Standardanalyten als auch den Analyten bindet. Der erste Fall ist stark bevorzugt.

In dem erfindungsgemäßen Verfahren ist es jedoch prinzipiell auch möglich, zwei oder mehr Sonden einzusetzen, die spezifisch fiir entsprechend viele unterschiedliche, zu bestimmende Analyten sind. Auch hier ergeben sich einige der erfindungsgemäßen Vorteile.

Ein wesentliches Merkmal der Erfindung ist die Tatsache, daß mindestens zwei der Sonden unterschiedliche Markierungsgruppen tragen, wobei eine ein durch elektrische Anregung und die andere ein durch chemische Anregung erzeugtes Lumineszenzsignal liefern kann. Im einfachsten Falle werden zwei Sonden eingesetzt, von denen jede eine unterschiedliche Markierungsgruppe trägt. Von diesen zwei Sonden ist eine spezifisch für den zu bestimmenden Analyten. In weiteren Fällen kann eine oder mehrere dieser Sonden mehrere identische Markierungsgruppen tragen, z. B. eine Sonde zwei gleiche Markierungsgruppen. Dies kann zu einer Erhöhung der Sensitivität beitragen. Es ist jedoch auch möglich, z. B. für die Bestimmung mehrerer Analyten mehr als zwei Sonden einzusetzen, welche dann mehr als zwei unterschiedliche Markierungsgruppen tragen. Auch hier kann jede Sonde ein oder mehrere gleiche Markierungsgruppen aufweisen. Vermieden werden sollte der Fall, daß eine Sonde zwei unterschiedliche Markierungsgruppen im Sinne der Erfindung trägt.

Die Sonden werden der Probe in einer Menge und Konzentration zugesetzt, die fiir eine ausreichende Bindung der Sonde an den Analyten, die Analyten oder den Standardanalyten geeignet ist. Da in vielen Fällen die Menge an Analyt nicht bekannt ist, wird die Sonde üblicherweise in einem stöchiometrischen Überschuß gegenüber der maximal denkbaren Menge an Analyt zugegeben. Dies gilt insbesondere, wenn eine quantitative Auswertung des Verfahrens beabsichtigt ist.

Hierzu ist es erfindungsgemäß nicht erforderlich, die Probe, nach Amplifikation, zu teilen und jedem Teil eine der Sonden zuzugeben. Bevorzugt werden die Sonden zusammen mit der Probe inkubiert, nicht getrennt. Daher sind relativ geringe Probenvolumina erforderlich.

Unter einer Markierungsgruppe im Sinne der Erfindung werden Gruppen verstanden, die direkt oder indirekt an die Sonden gebunden sein können. Darüber hinaus können die Markierungsgruppen ihrer Art nach in zwei Klassen eingeteilt werden, nämlich solche Gruppen, die selbst kein fiir eine Bestimmung ausreichendes elektromagnetisches Signal liefern können und Gruppen, die selbst ein elektromagnetisches Signal liefern können. Markierungsgruppen der letzten Definition werden im Folgenden auch als Detektionsgruppen bezeichnet. Gruppen, die selbst kein fiir eine Bestimmung ausreichendes elektromagnetisches Signal liefern können, sind bevorzugt alle über biologische Wechselwirkungen, wie sie oben für die Wechselwirkung der Sonde mit dem Analyt beschrieben sind, erkennbare Gruppen, z. B. Haptene, wie Digoxigenin gemäß EP-B-O 324 474, oder Vitamine, z. B. Biotin. Digoxigenin kann beispielsweise durch einen Antikörper gegen Digoxigenin und Biotin mit Hilfe von Avidin, Streptavidin oder Antibiotin/Antikörpem erkannt werden.

Für den Fall der indirekten Markierung ist im erfindungsgemäßen Verfahren die Verwendung eines Konjugates aus einer die genannte erkennbare Gruppe erkennenden Komponente (Antikörper, Avidin etc.) und einer Gruppe, die ein elektromagnetisches Signal liefern kann (Detektionsgruppe), bevorzugt. Dieses Konjugat kann der Inkubationsmischung der Probe mit den Sonden bereits zu Anfang der Inkubation zugegeben werden, bevorzugt ist jedoch die Zugabe des Konjugates nach erfolgter Inkubation der Sonden mit dem Analyten, ohne Abtrennung oder nach Abtrennung nicht an den Analyten gebundener analytspezifischer Sonde von der analytgebundenen Sonde.

Des weiteren hat es sich als höchst empfehlenswert erwiesen, nach Inkubation der Probe mit den Sonden nicht an den Analyten gebundene analytspezifische Sonde von der analytgebundenen Sonde sowie weitere an Probenbestandteile gebundene Sonden von den nicht gebundenen Sonden abzutrennen. Dasselbe gilt fiir die Abtrennung nicht gebundenen Konjugats. Diese Abtrennung kann vorteilhafterweise dadurch geschehen, daß die Bindeprodukte des Analyten bzw. der weiteren Probenbestandteile bzw. des Standardanalyten mit den zugehörigen Sonden an eine feste Phase (z. B. einen Festkörper in Form eines Beads) gebunden werden und die verbleibende flüssige Phase von der festen Phase getrennt wird. Dies kann beispielsweise geschehen durch Zurückhalten der festen Phase an einem Filter, während die Flüssigkeit durch das Filtermaterial durchtreten kann. Eine weitere Möglichkeit ergibt sich durch Verwendung magnetischer fester Phasen, und Anlegung eines Magnetfeldes, so daß sich die magnetischen Phasen an einer bestimmten Stelle sammeln und die Flüssigkeit mit den nicht gebundenen Sonden entfernt werden kann. Weiter bevorzugt wird die feste Phase mit Hilfe einer Waschflüssigkeit von physikalisch anhaftenden, nicht jedoch an den Analyten bzw. die anderen Bestandteile spezifisch gebundenen Sonden entfernt wird.

Die Bindung des Analyten an die feste Phase hängt wiederum von der Art des zu bestimmenden Analyten ab. So können wiederum biologische Wechselwirkungen (bevorzugt jedoch einer anderen Art oder/und Spezifität als bei der Bindung des Analyten bzw. Standardanalyten) für die Bindung ausgenutzt werden. Sofern es sich bei dem Analyten um ein Antigen handelt, kann die Bindung über eine feste Phase geschehen, die an ihrer Oberfläche durch Bindung eines Antikörpers gegen dieses Antigen modifiziert ist. Für den Fall von Nukleinsäuren als Analyten kann die Bindung über Sonden geschehen, die eine Nukleotidsequenz aufweisen, die komplementär zu einer Sequenz des Analyten, der weiteren Probenbestandteile bzw. der Standardnukleinsäure ist. Bevorzugt wird die Sequenz dieser sogenannten Fangsonde, die kovalent oder über biospezifische Wechselwirkungen, z. B. Biotin/Streptavidin, an die feste Phase gebunden ist oder wird, so gewählt, daß sie sowohl komplementär zu einer Teilsequenz des Analyten als auch der Standardnukleinsäure oder weiterer Analytnukleinsäuren ist. Dies ist insofern vorteilhaft, als dann für das Abfangen beider Probenbestandteile nur eine einzige Art von Fangsonde erforderlich ist. Ist der Analyt eine Zelle, so können diese Zellen durch Antikörper, die gegen Oberflächenantigene auf diesen Zellen gerichtet sind (z. B. CD3 für T-Zellen), immobilisiert werden. Die Selektion/Detektion kann mit Antikörpern gegen Untergruppen (z. B. der T-Helferzellen mit CD4 mit einer ersten Markierungsgruppe, der T-Supressorzellen mit CD8 und einer zweiten Markierungsgruppe) vorgenommen werden.

Eine weitere Möglichkeit der Bindung der Analytnukleinsäure bzw. der Standardnukleinsäure oder weiterer Analytnukleinsäuren an eine feste Phase ist der Einbau einer immobilisierbaren Gruppe in erzeugte Kopien während der Nukleinsäureamplifikation. Dies kann geschehen durch Verwendung immobilisierbar markierter Primer oder immobilisierbar markierte Mononukleosidtriphosphate. Als immobilisierbare Gruppe kann beispielsweise Biotin gewählt werden. Die erhaltenen Amplifikate können dann an einer streptavidinbeschichteten Oberfläche abgefangen werden.

Basierend auf der Anwesenheit der Markierungs- bzw. Detektionsgruppen wird nun ein elektromagnetisches Signal fiir jede unterschiedliche Markierungs- bzw. Detektionsgruppe erzeugt. Überraschenderweise hat es sich herausgestellt, daß die Kombination von auf unterschiedliche Weise anregbaren Gruppen für die unterschiedlichen Sonden besonders vorteilhaft ist. Vorteilhaft ist die erfindungsgemäße Kombination einer Sonde, die elektrisch zur Lumineszenz angeregt werden kann (Elektrochemilumineszenz, ECL), mit einer Sonde, die chemisch zur Lumineszenz angeregt werden kann (Biolumineszenz). Die selektive, d. h., gezielte Anregung der Signalbildung bewirkt beispielsweise, daß Kreuzanregung, d. h., Mitanregung auch der gerade nicht nachzuweisenden Markierungsgruppe stark reduziert oder sogar vermieden werden kann. Dies bewirkt für das Gesamtverfahren einen großen dynamischen Meßbereich und relativ niedrige Hintergrundsignale (hohe Sensitivität). Die Art der Erzeugung hängt von den jeweiligen Erfordemissen im Hinblick auf die Signalerzeugung ab. Eine erste Gruppe von Markierungsgruppen zeichnet sich dadurch aus, daß das Signal durch Inkontaktbringen der Markierungsgruppe mit bestimmten (z. B. chemischen) Reagenzien erzeugt wird. Eine bevorzugte Gruppen von Detektionsgruppen sind zur Biolumineszenz anregbare Gruppen, z. B. allosterisch triggerbare Gruppen. Die Detektionsgruppen unterschiedlicher Sonden gehören bevorzugt unterschiedlichen Substanzklassen an (z. B. ein niedermolekularer organischer Metallkomplex und ein Protein). Zu Markierungsgruppen gehören beispielsweise die durch Ionen aktivierbaren Photoproteine (Apo-Photoproteine, nativ oder bevorzugt recombinant hergestellt). Zu diesen Photoproteinen gehören beispielsweise Aequorin, Obelin, Mitrocomin, Thalassicolin und Clytin. Diese Proteine haben die Eigenschaft, daß sie ein Lichtsignal abgeben, wenn sie aktiviert werden, so daß ihre Menge oder Anwesenheit durch Messung der Lichtmessung bestimmt werden kann. Die Verwendung solcher Photoproteine in herkömmlichen Tests und der Mechanismus, der zur Signalbildung führt, ist beispielsweise in Cormier, M.L. et al., Photochem & Photobiol. 49/4, 509 - 512 (1989) oder Smith, D.F. et al., in "Bioluminescence and Chemiluminescence: Current Status (P. Stanley & L. Kricka, eds.), John Wiley and Sons, Chichester, U.K. 1991, 529 - 532 beschrieben. In diesem Fall findet die Aktivierung durch Zugabe von Kalziumionen zu den markierten, gebundenen Sonden statt.

Adequate Chemilumineszenzdirektmarkierungen mit blitzartigem Signalprofil sind z. B. auch Acridinium-Arylester, Acridinium-Acyl-Sulfonamide oder Isoluminolderivate, wie ABEI,

AEEI und AHEI.

Eine weitere, besonders geeignete Gruppe von Markierungsgruppen sind zur Elektrochemiluminszenz anregbare Metallkomplexe. Solche Komplexe sind beispielsweise beschrieben in EP-A-0 199 804, EP-A-0 265 519, WO 89/04302 und WO 92/14139. Bevorzugt handelt es sich um Rutheniumkomplexe, welche als Liganden Bipyridyleinheiten aufweisen. Nukleinsäuresonden, die mit speziellen Rutheniumhomplexen markiert sind, sind beispielsweise auch in EP-A-0 340 605 beschrieben. Besonders gut einsetzbare immunologische Sonden, welche mit Rutheniumkomplexen markiert sind, sind beispielsweise in EP-A-0 178 450 beschrieben. Neben Rutheniumkomplexen können auch andere entsprechend triggerbare Übergangsmetalle (Osmium, Iridium, etc.) bzw. andere zur Elektrochemilumineszenz geeignete heterocylisch aromatische Komplexliganden verwendet werden.

Im erfindungsgemäßen Verfahren werden die Detektionsgruppen zu einer Bildung eines detektierbaren, auf die Gruppe zurückzuführenden Signals, angeregt. Bevorzugt ist dieses Signal ein Lumineszenzsignal. Unter Lumineszenz wird üblicherweise ein Vorgang verstanden, bei dem ein chemisch angeregter, metastabiler Molekülzustand unter Emission eines Photons (elektromagnetische Strahlung) in einen niederenergetischen Zustand übergeht

Unter unabhängigen Erzeugungen eines Signals werden im Sinne vorliegenden Erfindung Handlungen verstanden, durch die selektiv eine bestimmte Markierungsgruppe angeregt wird, ohne daß Markierungsgruppen auf davon unterschiedlichen Sonden angeregt werden.

Unter einem elektromagnetischen Signal wird im Sinne der Erfindung bevorzugt ein Lichtsignal verstanden. Besonders bevorzugt sind Lichtblitze.

Von unterschiedlichen elektromagnetischen Signalen wird im folgenden gesprochen, wenn sich die Signale in ihrer Herkunft unterscheiden, z.B. herkommend von unterschiedlichen Substanzklassen von Markierungsgruppen. Wenn im Folgenden von der Kombination von Elektrochemilumineszenz und Biolumineszenz durch Photoproteine gesprochen wird, so ist dies nur als bevorzugte Ausführungsform eines Systems zweier unterschiedlich anregbarer Lumineszenzmarkierungen zu verstehen.

Die Erzeugung der unterschiedlichen elektromagnetischen Signale kann sowohl gleichzeitig als auch nacheinander vorgenommen werden. Dies hängt insbesondere davon ab, ob die Signale so stark unterschiedlich sind, daß sie auch gleichzeitig spezifisch detektiert werden können. Es hat sich jedoch als zweckmäßig erwiesen, die unterschiedlichen elektromagnetischen Signale nacheinander zu erzeugen und zu detektieren. Daher ist es bevorzugt, Markierungsgruppen zu wählen, die selektiv aktivierbar sind, z. B. eine erste Gruppe durch chemische oder/und allostenische Triggerung und zweite durch elektrochemische Triggerung. Dies hat insbesondere den Vorteil, daß die für die Signalerzeugung eventuell erforderlichen Reaktionsbedingungen nicht in derselben Lösung eingestellt werden müssen. Für den Fall, daß es sich bei der ersten Art von Markierungsgruppe um ein durch Ionen aktivierbares Photoprotein und bei der zweiten Markierungsgruppe um einen zur Elektrochemilumineszenz anregbaren Metallkomplex handelt, ist es möglich, die beiden Signale gleichzeitig oder nacheinander zu bestimmen. Eine gleichzeitige Messung ist möglich bei Verwendung von Markierungsgruppen mit unterschiedlichen Emissionswellenlängen. Das zur Lichtdetektion verwendete Modul muß dabei eine spektroskopische Auftrennung der Signale, z. B. durch Wahl geeigneter Filter, Prismen oder

Gitter erlauben.

Bei Bestimmung der Signale nacheinander ist es möglich, zuerst die Bedingungen für die Erzeugung des Photoproteinsignales einzustellen, aber bevorzugt, zunächst die Bedingungen für die Erzeugung der Elektrochemilumineszenz einzustellen. Hierzu wird (für das Beispiel der Markierung mit einem Rutheniumtrisbipyridylkomplex) eine Lösung von Kaliumphosphat, Tripropylamin und Thesit™ mit den an die feste Phase gebundenen Analyten, Standardanalyten und gegebenenfalls anderen zu bestimmenden Bestandteilen, an welche die Sonden gebunden sind, in Kontakt gebracht. Dies kann entweder durch Zupipettieren der Lösung zu der festen Phase geschehen, es ist jedoch auch möglich, die feste Phase in eine Lösung der Reagenzien zuzugeben oder die Lösung an der festen Phase vorbeifließen zu lassen. Der letzte Fall ist bevorzugt, da hierbei auch eine Reinigungswirkung erzeugt wird. In Anwesenheit der zwei oder mehr Sonden wird nun die Elektrochemilumineszenz durch Anlegen einer Spannung erzeugt. Der erzeugte Lichtblitz wird als Signal mit Hilfe eines geeigneten Instrumentes, z. B. unter Anwendung eines Photomultipliers, in einen Meßwert umgewandelt. Für die Meßbedingungen und Reagenzien wird auf die Publikation Uland, J.K. and Powell, M.J. in J. Electrochem. Soc. 137, pp. 3127-3127 (1990) verwiesen. Überraschenderweise stört die elektrische Signalerzeugung die Aktivität der Photoproteine nicht.

Anschließend werden Reaktionsbedingungen eingestellt, wie sie für die Erzeugung eines Signales basierend auf der Anwesenheit des Photoproteins erforderlich sind. Auch hier können die entsprechenden Reagenzien zu der bereits vorliegenden Lösung oder einer entsprechenden Waschlösung oder der festen Phase zugegeben werden. Bevorzugt ist jedoch die Verdrängung der fiir die Erzeugung der Elektrochemilumineszenz erforderlichen Reagenzlösung durch die Reagenzlösung für die Aktivierung des Photoproteins. Das Inkontaktbringen des Photoproteins mit dem aktivierenden Ion bewirkt ebenfalls die Erzeugung eines Lichtsignals, dessen Intensität instrumentell bestimmt und in einen Meßwert umgewandelt werden kann. Es war möglich, das Signal, welches mit Hilfe von Aequorin erzeugt worden war, mit derselben apparativen Anordnung wie der für die Detektion des Lichtblitzes, welcher mit Hilfe von Rutheniumbipyridylkomplexen erzeugt worden war, zu messen, vorausgesetzt der Photomultiplier hat eine genügende Empfindlichkeit bei beiden Emissionswellenlängen. Für die Bedingungen zur Erzeugung eines Lichtsignales bei Verwendung von Aequorin als Label wird hiermit Bezug genommen auf die Publikation Smith, D.F. et alt Bioluminescence and Chemiluminescence; Current status (P. Stanley and L. Kricha, eds.), John Wiley, Chichester, U.K., pp. 529-532 (1991).

Bei den Signalen handelt es sich bevorzugt und vorteilhafterweise um Blitzsignale. Dies bedeutet, daß Anregung und Messung des Signals sehr schnell ablaufen können. Dadurch wird die Gesamtmeßzeit für zwei Nachweisreaktionen nicht wesentlich länger (≥ 2,5 x) als die fiir eine der beiden Einzelmessungen (z. B. Elektrochemilumineszenz: 4 Sekunden, Elektrochemilumineszenz mit nachfolgender Biolumineszenz mit Aequorin: 5 Sekunden). Dies ermöglicht einen maximalen Zugewinn bezüglich des Durchsatzes, besonders auf einem Analyzer, da das Prinzip des dualen Markierungsnachweises nicht gemindert wird durch eine signifikante Aufweitung des Meßzeitfensters. Es ergeben sich für die genannte Kombination keine Interferenzen zwischen den beiden einer Probe zugeordneten Signalreaktionen durch vollständige zeitliche Trennung innerhalb eines Meßschrittes, so daß eine saubere, voneinander unabhängige Auswertung der beiden Signale direkt möglich wird, ohne Notwendigkeit für letztlich nur Nährungswerte liefernde mathematische Korrektoralgorithmen. Die jeweilige Leistungsfähigkeit der beiden Markierungsgruppen (z. B. im Hinblick auf Kriterien, wie analytische Sensitivität, Serien- und Tag-Tag-Präzision, dynamischer Meßbereich und linearer Meßbereich) bleibt auch in Kombination erhalten.

Die Messung der von unterschiedlichen Detektionsgruppen erzeugten Signale erfolgt bevorzugt mit derselben Messanordnung, z. B. in derselben Messzelle. Bevorzugt wird die Messzelle so gewählt, daß an ihr keine Änderungen bezüglich der detektierten Wellenlängen für beide Detektionsgruppen erforderlich ist. Die Messzelle ist bevorzugt eine für das elektrochemische Triggern einer Detektionsgruppe ausgelegte Messzelle. Die bevorzugt nachgeschaltete Detektion beruhend auf einer anderen Substanzklasse als Detektionsgruppe benutzt nicht die Elektroanordnung der ECL-Messzelle zur Anregung der Lumineszenz, sondern nur zur Detektion.

Die sequentielle Abfolge der Bestimmung der unterschiedlichen Detektionsgruppen macht es moglich, daß die Gruppen keine unterschiedliche kinetische Charakteristik aufweisen müssen, oder sich in ihrem spektralen Verhalten unterscheiden müssen. So ist beispielsweise der Ruthenium-Komplex durch einen Fluorophor ersetzbar, der wie Aequorin in blaugrünem Bereich emittiert.

Die Auswertung der Signale bzw. der Meßwerte für diese Signale hängt nun von der beabsichtigten Aussage des Bestimmungsverfahrens ab. In jedem Fall jedoch ist das Vorhandensein eines Meßwertes, der sich von einem Meßwert einer Vergleichsmessung unterscheidet, bei der keine Sonde oder kein Analyt vorhanden war, ein Anzeichen für die Anwesenheit des Analyten. Dasselbe gilt fiir den Meßwert für die übrigen Bestandteile oder einen Standardanalyten. Dies kann benutzt werden, um eine qualitative Aussage über die Anwesenheit des Analyten bzw. weitere Analyten zu erhalten. Das Ergebnis der Messung für den Standardanalyten kann in einer qualitativen Messung für die Eichung des Systems und als Positivkontrolle verwendet werden.

Eine bevorzugte Ausführungsform des erfindungsgemäßen Verfahrens enthält die Schritte:
- Bindung des Analyten und gegebenenfalls eines weiteren Analyten bzw. Standardanalyten an eine feste Phase (z. B. ein Magnetpartikel),
- Bindung einer Sonde an den Analyten bzw. an den weiteren Analyten bzw. den Standardanalyten,
- Bestimmung der gebundenen ersten Sonde (in Anwesenheit, jedoch unabhängig von der zweiten Sonde),
- Bestimmung der Bindung der zweiten Sonde (in Anwesenheit und unabhängig von der ersten Sonde),
- Ermittlung der Anwesenheit bzw. Konzentration des mindestens einen Analyts aufgrund der erhaltenen Signale der ersten Sonde. Hierbei können jedoch auch weitere Sonden, markiert mit weiteren Markierungsgruppen und unter Verwendung weiterer unabhängiger Anregungen, eingesetzt werden. Bevorzugt wird der Analyt, der weitere Analyt bzw. Standardanalyt und die daran gebundenen Sonden nach Durchführung der Bestimmung vom Anregungsbzw. Meßort entfernt, so daß dieser für die Durchführung einer weiteren Bestimmung, z. B. der Bestimmung eines Analyten aus einer weiteren Probe, zur Verfügung steht.

Das erfindungsgemäße Verfahren ist jedoch besonders geeignet fiir eine quantitative Bestimmung eines Analyten. Unter einer quantitativen Bestimmung wird die Bestimmung der Menge eines Analyten in der Probe und somit auch die Bestimmung der Menge an Analyt in einer für die Herstellung der Probe verwendeten Primärprobe verstanden. Die quantitative Bestimmung verwendet bevorzugt mindestens eine Sonde, die für den zu bestimmenden Analyten spezifisch ist. Sollen mehrere Analyten bestimmt werden, sind weitere Sonden/Markierungsgruppen erforderlich. Darüber hinaus wird eine Sonde eingesetzt, welche fiir den Standardanalyten spezifisch ist. Da die Menge an Standardanalyt definiert und bekannt ist, kann der Meßwert fiir den Standardanalyt zur Eichung des Meßwertes fiir den Analyt verwendet werden.

Die Eichung des Systems kann z. B. folgendermaßen durchgeführt werden:
I. Erstellung einer Eichkurve:
   Verschiedene Lösungen, z. B. 5, mit unterschiedlichen, aber bekannten Konzentrationen an Analytnukleinsäure, werden mit jeweils der gleichen bekannten Menge an Standardnukleinsäuren (z. B. 1000 Kopien) versetzt. Zu jeder dieser Lösungen werden Lösungen mit den mindestens 2 Sonden (z. B. Rutheniummarkierung an der Analytsonde, Aequorinmarkierung an der Standardsonde) gegeben. Nach Hybridisierung werden die Meßsignale für die bekannten Konzentrationen an Analyt und Standard ermittelt. Daraus wird eine Eichkurve erstellt, indem das Signalverhältnis der Messungen für Analyt zu Standard in Abhängigkeit der eingesetzten bekannten Analytkonzentration aufgetragen wird.
II. Probenmessung und Auswertung
   Der Probe mit der unbekannten Analytkonzentration wird vor der Messung die gleiche Konzentration an Standardnukleinsäuren wie bei der Erstellung der Eichkurve (z. B. 1000 Kopien) zugegeben und die entsprechenden Meßsignale für den Analyt und den Standard ermittelt. Das Signalverhältnis von Analyt zu Standard wird berechnet und die Analytkonzentration aus der Eichkurve abgelesen.
   Die Verhältnisbildung aus 2 oder mehr gemeinsam (d. h. aus einem Reaktionsansatz) erzeugten Signalen ist eine einfache Möglichkeit, eventuell auftretende Schwankungen in der Signalgenerierung zu kompensieren. Vorallem werden eventuelle Schwankungen in der Amplifikationsreaktion der unterschiedlichen Sonden für den Analyten bzw. Standard korrigiert. Solche Schwankungen betreffen z. B. variable Effizienzfaktoren von Probe zu Probe, oder für ein und dieselbe Probe von Cyclus zu Cyclus und rühren unter anderem von Schwankungen in der Qualität der Probenvorbereitung her (z. B. Aufreinigungsgrad, Abtrennung von Inhibitoren). per internem, co-amplifiziertem Standard und ratiometrischer Auswertung werden solche Schwankungen für jeden Reaktionsansatz herausgerechnet. Sollte sich das Signalverhältnis zwischen den einzelnen Geräten und über die Zeit pro Gerät ausreichend konstant verhalten, kann dieses Signalverhältnis beim Hersteller des Gerätes bzw. der Reagenzien vorab ermittelt werden und zur Auswertung mittels Korrekturfaktor bzw. Korrekturfunktion in der Auswertesoftware implementiert werden. Dann ist kundenseitig nur noch die Erstellung einer Eichkurve aus verschiedenen Konzentrationen an Analytnukleinsäure notwendig.
III. Vor Erstellung der Eichkurve erfolgt zweckmäßigerweise eine experimentelle Prüfung auf vergleichbare Amplifikationseffizienz von Proben-DNA und Standard-DNA (interner Standard), erkennbar z. B. an parellel-linearen Verläufen bei Auftragung von log Signal (Y) über log Ausgangskopienzahl N₀ (X)

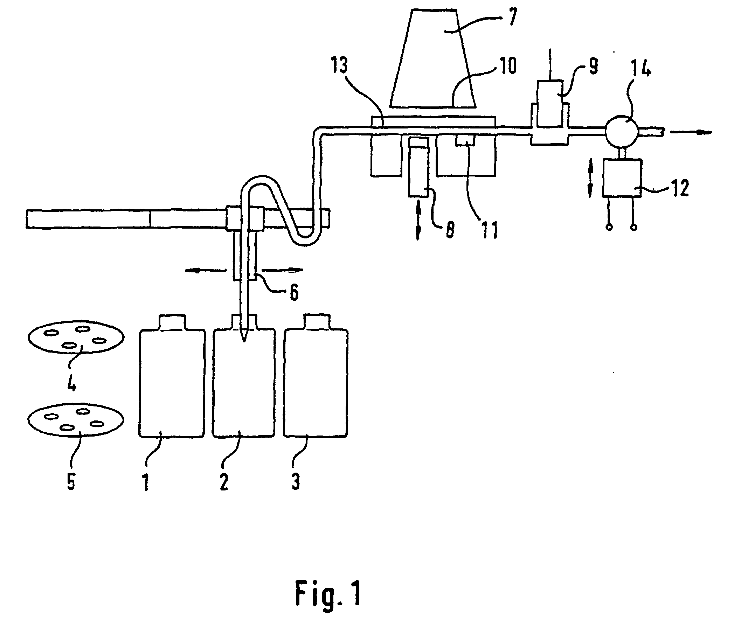

   In Figur 1 ist der schematische Aufbau eines Gerätes zur Durchführung des erfindungsgemäßen Verfahrens gezeigt. Ein entsprechendes Gerät, das einfach auf die Anforderungen des beschriebenen Verfahrens adaptiert werden kann, ist von der Firma Boehringer Mannheim GmbH (Elecsys 2010 oder 1010) erhältlich.

Bevorzugt laufen folgende Schritte ab:
1. Amplifikation mittels PCR einer Analytnukleinsaure in einer Probe in Anwesenheit und Coamplifikation einer bekannten Menge an Standardnukleinsäure.
2. Denaturierung durch Zugabe von NaOH-Lösung.
3. Aufbewahrung dieser Probe im Probenrotor 5.
4. Überführung eines Aliquots in ein Gefäß des Inkubators.
5. Zugabe der analytspezifischen und der standardanalytspezifischen Sonde.
6. Inkubation mit streptavidinbeschichteten Magnetpartikeln in Inkubator 4.
7. Aufnahme eines Aliquots der Lösung aus dem Inkubator 4 über die Pipettiernadel 6 in die Meßzeile 13.
8. Aufnahme der Konditionierlösung aus Behälter 1 zum Transport des Aliquots durch das Liquid Flow System. Die Magnetteilchen mit gebundenen Analyt, Standardanalyt und Sonden werden durch den Magneten an der Arbeitselektrode festgehalten.
9. Aufnahme von Konditionierlösung aus Behälter 1 zum Waschen der Beads auf der Arbeitselektrode.
10. Anlegen einer Spannung zwischen Arbeits- und Gegenelektrode 10 und 11 (kontrolliert über Referenzelektrode 9) führt zur Aussendung eines Lichtblitzes. Messung des Lichtblitzes mit dem Photomultiplier 7.
11. Aufsaugen der kalziumhaltigen Triggerlösung aus Gefäß 3 in die Meßzelle 13, dabei Messung der entstehenden Lumineszenz. Die Teilchen sind hierbei durch den Magneten 8 in der Meßzeile 13 festgehalten.
12. Entfernen des Magneten 8 und somit Abführung der gebundenen Magnetteilchen mit durchgepumpter Reinigungslösung aus Gefäß 2.
13. Auswertung der Signalintensitäten.
14. Das Gerät steht zur erneuten Aufnahme einer zu vermessenden Probe (Schritt 1) zur Verfügung.

Der Gegenstand der vorliegenden Erfindung kann in vielerlei Hinsicht vorteilhaft genutzt werden. Zunächst ist das Verfahren für die Durchführung quantitativer Bestimmungen von Analyten geeignet. Die Menge an benötigter Probenflüssigkeit wird reduziert, gegebenenfalls halbiert. Bei Verwendung der bevorzugten Detektionsmarkierungen ist nur ein einziges Gerät zur Detektion und Auswertung erforderlich. Außerdem ist es möglich, auch immunologische Tests mit auf Nukleinsäurebasis funktionierenden Tests zu kombinieren. Auf jeden Fall wird der Durchsatz von Proben auf Testgeräten (Analyzer) ganz erheblich verbessert. Durch unabhängige Anregung wird die zeitliche Trennung der Signale ermöglicht. Dies ermöglicht einen großen dynamischen Bereich und die hohe Sensitivität.

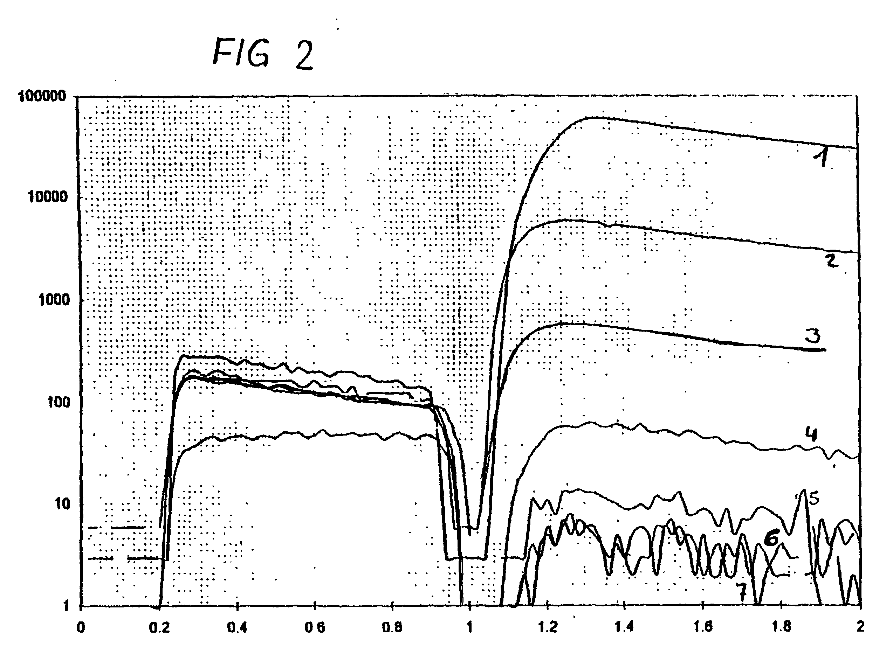

In Figur 2 ist der Signalverlauf einer beispielhaften, hintereinander ablaufenden Bestimmung zweier Detektionsgruppen (Rutheniumkomplex und Aequorin) gezeigt. Es wird erkenntlich, daß die Signalintensitaten in der gleichen Größenordnung liegen, d. h. überraschenderweise auch für Aequorinlabel in eigentlich zur Bestimmung der Elektrochemilumineszenz gedachten Geräten eine ausreichende Signalausbeute erreicht werden kann. In Figur 2 bedeuten die Kurvenverläufe 1 bis 7:

| | |
|---|---|
| 1 | 10 nM bio-Aeq /7 pM ruthenyliertes Oligonukleotid |
| 2 | 1 nM bio-Aeq / 7 pM ruthenyliertes Oligonukleotid |
| 3 | 100 pM bio-Aeq / 7 pM ruthenyliertes Oligonukleotid |
| 4 | 10 pM bio-Aeq /7 pM ruthenyliertes Oligonukleotid |
| 5 | 1 pM bio-Aeq / 7 pM ruthenyliertes Oligonukleotid |
| 6 | 0 M bio-Aeq / 7 pM ruthenyliertes Oligonukleotid |
| 7 | 0 M bio-Aequorin /0 M ruthenyliertes Oligonukleotid |

Aufgetragen ist die Signalstärke bei Vermessung gegen die Zeit in Sekunden.

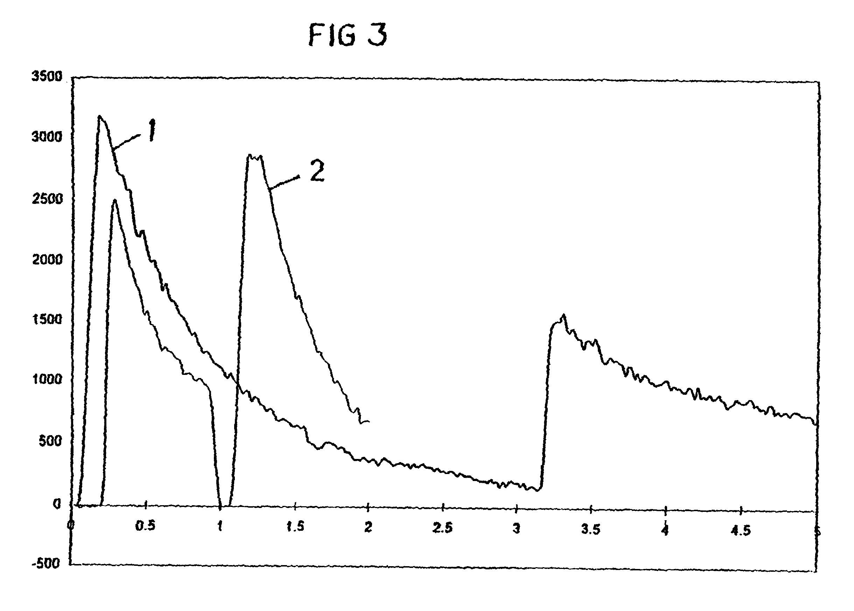

Figur 3 zeigt, daß überraschenderweise praktisch kein Signalverlust durch vorgeschaltete ECL-Messung zu beobachten ist. In Figur 3 ist der Verlauf der Signalintensitäten (bei gleichen Konzentrationsverhältnissen) während einer Meßzeit in Sekunden gezeigt fiir einen ersten Fall, bei dem zuerst das Aequorinlabel von MDP (Mono-biotin-Mono-DigoxigeninHepta-Peptid, erhältlich aus dem Enzymun-Test® DNA Detection, Boehringer Mannheim GmbH, BRD, Best.-Nr. 1447777) und anschließend das ECL-Signal des Oligonukleotids 1 (SEQ.ID.NO. 1) gemessen wird (Kurve 1) und einem zweiten Fall, in dem zuerst das ECL-Signal und anschließend das Aequorinsignal gemessen wird (Kurve 2). Es ist erkennbar, daß sich die Intensität des Aequorinsignals vor ECL-Messung nur unwesentlich von der Intensität des Signals nach ECL-Messung unterscheidet. Dies bedeutet, daß die Biolumineszenzmarkierung überraschenderweise durch den vorgeschalteten Elektrochemielumineszenzprozess nicht wesentlich an Funktionalität verliert. Die Triggerspannung für die ECL-Messung ist jenseits der Zersetzungsspannung von Wasser, d.h., die Wasseroxidation ist während des ECL-Prozesses in vollem Gange, an der Anode (wo sich das Äquorin befindet), entstehen große Mengen Sauerstoff. Die Umgebung des Proteins wird also erheblich gestört. Überraschenderweise wird dadurch die durch das Protein hervorgerufene Signalhöhe nicht beeinflußt. Es muß weiterhin gewährleistet werden, daß Reste der Reaktionslösungen für die vorangegangene Bestimmung nicht mehr in störendem Umfang in der Messzelle verbleiben. Das gilt insbesondere für Substanzen (z. B. Ionen), die, wenn die beiden Lösungen für die unterschiedlichen Reaktionen aufeinandertreffen, schwerlösliche Niederschläge bilden (z. B. Calziumionen aus der Aequorintriggerung und Phosphationen aus der ECL-Triggerung). Gerade die Verwendung unterschiedlich und vollständig voneinander getrennt anregbarer Markierungen in nacheinander ablaufenden Bestimmungsreaktionen insbesondere fiir Analysenautomaten, bei denen eine Vielzahl von Bestimmungen nacheinander durchgeführt werden soll, und wobei die später durchgeführten Bestimmungen noch genauso zuverlässig durchgeführt werden sollen, wie die ersten, war für einen Fachmann nicht zu erwarten. Dies gilt insbesondere für Analysenautomaten mit Durchflußküvetten, welche für unterschiedliche Bestimmungen eingesetzt werden.

Gegenstand der Erfindung ist auch ein Reagenzkit zur Bestimmung eines Analyten enthaltend in einem oder getrennten Behältern zwei oder mehr Sonden, von denen mindestens eine für den zu bestimmenden Analyten spezifisch ist, wobei von den mindestens zwei Sonden eine elektrisch zur Lumineszenz und eine chemisch zur Lumineszenz angeregt werden kann.

Die Spezifität der weiteren Sonden ist der obigen Schilderung des erfindungsgemäßen Verfahrens zu entnehmen. Darüber hinaus enthält das Reagenzkit bevorzugt einen Behälter mit einem Standardanalyten, wobei die Konzentration des Standardanalyten bekannt oder/und definiert ist. Außerdem kann das Reagenzkit weitere Reagenzien, die zur Bestimmung der Markierungsgruppen erforderlich sind, enthalten. Weiterhin können auch Reagenzien zur Vorbehandlung von Proben zur Herstellung einer analythaltigen, fiir die Bestimmung geeigneten Lösung, enthalten sein. Diese sind bevorzugt in getrennten Behältern enthalten. Geeignete Reagenzien sind beispielsweise Primer, Enzym- und Mononukleosidtriphosphate für die Durchführung einer kompetitiven PCR. Weitere mögliche Bestandteile des Reagenzkits sind eine Suspension von Magnetpartikeln, an welche der Analyt gebunden werden kann.

Weiterer Gegenstand der Erfindung ist die Verwendung zweier Sonden, gemäß Anspruch 14 zum quantitativen Nachweis von Analyten in einer Probe.

Die folgenden Beispiele sollen den Gegenstand der Erfindung näher erläutern:

### Beispiel 1

### Aufeinanderfolgende Bestimmung von unterschiedlichen Detektionsgruppen an streptavidinbeschichteten Magnetpartikeln bei gleichzeitiger Anwesenheit von Rutheniumkomplexen als zweiter Detektionsgruppe

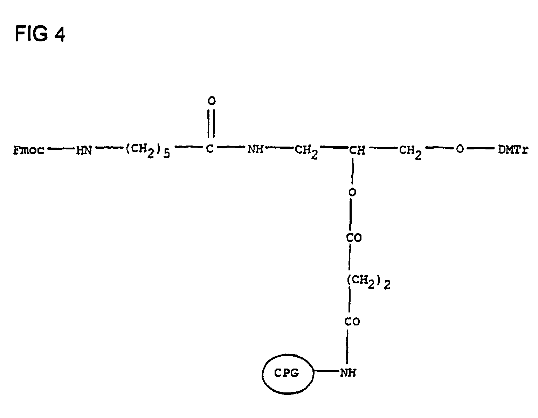

Für die Bestimmung unterschiedlicher Detektionsgruppen wurde eine 200 µl Pufferlösung hergestellt, die biotinyliertes Aequorin (in den Konzentrationen 1 pM bis 10 nm) und das am 5'-Ende mit Bio-Link I (Firma Applied Biosystems Inc., Biotin Amidite, Best.-Nr. 401395) biotinylierte und am 3'-Ende über AM III (Einbau während der Oligonukleotidsynthese an CPG (Controlled Pore Glass) und Entfernen der Schutzgruppe Fmoc, FIG 4) mittels BPRu (hergestellt gemäß Clin. Chem. 37/9, 1534 - 1539 (1991)) markierte Oligonukleotid I (SEQ.ID.NO. 1) enthält. Diese Probelösungen wurden mit 36 µg Streptavidinbeads (Firma Boehringer Mannheim GmbH, Elecsys® TSH Immunoassay, Best.-Nr. 1731459) in 50 µl des oben genannten Puffers versetzt und 5 min. bei 37 °C inkubiert. Anschließend wurden 120 µl dieser Reaktionslösung in die Meßzelle des Elecsys 2010- oder Elecsys 1010-Analyzers der Firma Boehringer Mannheim GmbH aufgenommen. Danach wurden 1100 µl einer Konditionierlösung mit der Zusammensetzung 300 mM Kaliumphosphat, 180 mM Tripropylamin (TPA) und 1,7 mM Thesit® durchgepumpt. Dann wurde ein Elektrochemilumineszenz(ECL)-Signal generiert (Spannung: 1,25 V, Zeit: 0,8 sek.). Das ECL-Signal wurde aufgenommen und ist auf dem linken Teil von Figur 2 zu sehen. Durch die konstante Konzentration des Rutheniumlabels erhält man bei allen ECL-Messungen das ungefähr gleiche Signal.

Zur Messung des Aequorinsignals wird eine Triggerlösung der Zusammensetzung 10 mM Tris/HCI, pH 7,4, 100 mM Calciumchlorid in die Meßzelle eingesaugt und dabei das entstehende Signal gemessen. Das gemessene Signal ist auf dem rechten Ast der Kurven in Figur 2 gezeigt. Es ist erkennbar, daß das Signal der Aequorinmessungen von der Konzentration der aequorinmarkierten Verbindungen abhängig ist.

### Beispiel 2

### Nachweis zweier verschiedenen Chlamydia - Targetsequenzen

Die reale Situation eines Dual Label Assays unter Benutzung eines internen Standards sieht so aus, daß eine interne Standardnukleinsäure bekannter Konzentration in Anwesenheit einer Analytnukleinsäure unbekannter Konzentration nachgewiesen werden muß. Dabei erstreckt sich der potentielle Konzentrationsbereich der Analytnukleinsäure über mehrere Größenordnungen.

Für eine gleichbleibend gute Quantifizierungsmöglichkeit über den gesamten Konzentrationsbereich ist es notwendig, daß das Standardsignal unabhängig von der Konzentration der Probe konstant bleiben muß und das Analytsignal linear mit der Probenkonzentration variiert, unabhängig von der Konzentration der ebenfalls vorhandenen Standardnukleinsäure.

Im vorliegenden Beispiel wird die Unabhängigkeit beider Signale mittels parallel geführter Bindereaktionen zwischen zwei verschiedenen Fangsonden (Standard bzw. Analyt) und zwei jeweils hybridisierungsfähigen Indikatorsonden (standard- bzw. analytspezifisch), von denen eine mit Rubpy, die andere Aequorin-markiert ist. Die reale Test-Situation wird dadurch modelliert, daß die Konzentration jeweils einer Fangsonde konstant gehalten wird (diese simuliert dann den Standard), während die der anderen Fangsonde ( die dann den Analyten simuliert) über vier Größenordnungen variiert wird.

Bei den Fangsonden handelt es sich um biotinylierte 40mere (20mer- (bzw. 19mer-) (nichtchlamydiaspezifische) Spacersequenz + 20-mer- (bzw. 21-mer-) Hybridisierungssequenz). Beide Hybridisierungssequenzen entstammen dem *Chlamydia trachomatis* Genom. Im einen Fall hat die Hybridisierungszone die Sequenz 5'-CA GAG TTC TAT AGT GCT ATG-3' (SEQ.ID.NO. 2). Die zugehörige Indikatorsonde 5'CAT AGC ACT ATA GAA CTC TG-3' (SEQ.ID.NO. 3) ist Aequorin-geiabelt (MH:SH Linkerchemie nach Funktionalisierung mit basisch aktivierbarem [N-Trifluoroacetamido]-aminoalkyl-phosphoramidit).

In der anderen Fangsonde ist die Hybridisierungssequenz 5'-G TCT CTC ATC GAG ACA AAG TG-3' (SEQ.ID.NO. 4). Die zugehörige Indikatorsonde 5'CA GAG TTC TAT AGT GCT ATG-3' (SEQ.ID.NO. 5) ist über NHS-Chemie mit Ru(bipy)₃ gekoppelt.

Von den beiden Chlamydia-Targets ( Fangsonde ) wird jeweils eines konstant bei 0.1nM gehalten, während die Konzentration des anderen seriell auf 1*10⁻⁹, 1*10⁻¹⁰, 1*10⁻¹¹, 1*10⁻¹² M verdünnt wird. Die Konzentration der beiden Indikatorsonden ist konstant (10nM). Die bei 37°C gebildeten Hybride werden auf Streptavidin-beschichteten ECL-Beads (720 µg/ml aus Elecsys® TSH) immobilisiert und anschließend mit dem Dual Label Zyklus wie in Beispiel 1 beschrieben vermessen.

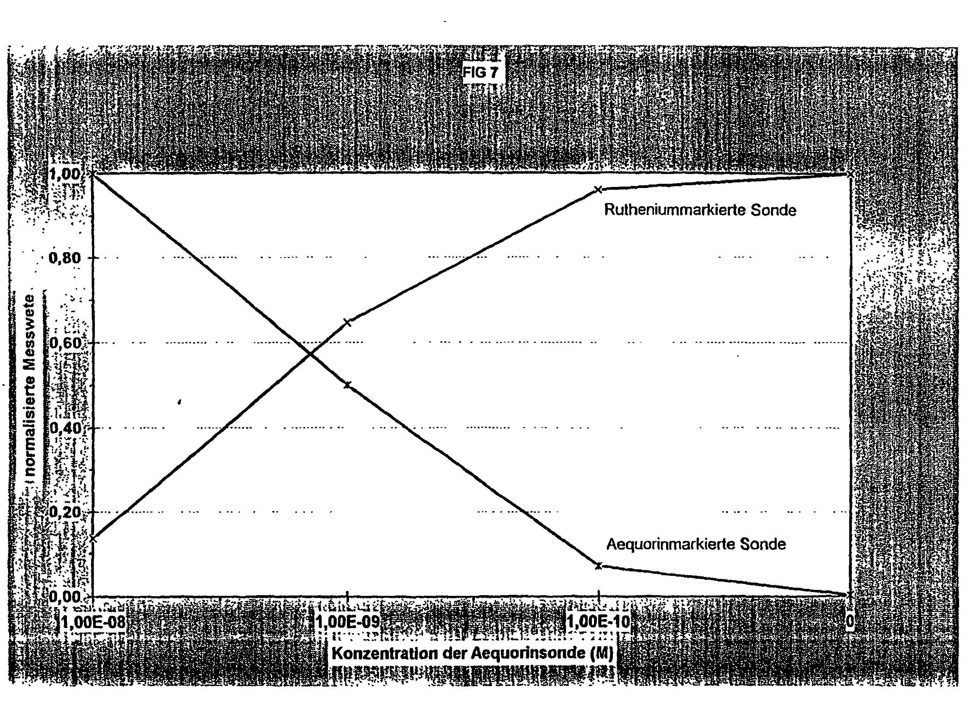

FIG 5 und 6 zeigen daß während das Signal des jeweils gewählten Standards konstant bleibt, sich für die jeweils als Analytnukleinsäure gewählte Fangsonde eine ausgezeichnete lineare Korrelation zwischen Analytkonzentration und -signal über vier Größenordnungen hinweg ergibt, unabhängig vom Signal des Standards.

### Beispiel 3

### Kompetitiver Assay

In diesem Versuch erfolgt eine competitive Testführung mit dual label-Detektion als Modellassay im Hinblick auf quantitative PCR (qPCR) via Co-amplifikation eines internen Standards, wobei ein Label (eine Markierung) definitiv einem zu amplifizierenden Polynucleotid zugeordnet ist. Hierbei repräsentieren die Indikatorsonden die per Konkurrenz um Primer-Anbindung variable Menge an detektierbarem PCR-Produkt, ausgehend von Analyt-DNA bzw. interne Standard-DNA (Hybridisierungsrate als Funktion der jeweiligen Ausgangskopienzahl).

### a. Versuchsaufbau:

Eine biotinylierte 30-mer Fangsonde (10 dT-Spacersequenz + 20-mer Hybridisierungszone: 5'-CA GAG TTC TAT AGT GCT ATG-3' (SEQ.ID.NO. 6)) wird seriell verdünnt auf 1000, 100, 10 pmol/l (dazu 0 pmol/l = Pufferleerwert) und jeweils auf Streptavidin (SA)-beschichteten ECL-Beads (720 µg/ml) immobilisiert. Eine dazu in ihrer Basensequenz komplementäre 20-mer Indikatorsonde (5'-CAT AGC ACT ATA GAA CTC TG-3' (SEQ.ID.NO. 7)), anknüpfend an eine Funktionalisierung mit basisch (NH₃) aktivierbarem [N-Trifluoracetamido]-aminoalkyl-phosphoramidit einmal über MH:SH-Linkerchemie mit Aequorin markiert, und einmal über NHS-Chemie mit Ru²⁺(bipy)₃, wird mit der Fangsonde zur Reaktion gebracht, wobei es zu einer Konkurrenz zwischen Aequorin- und ECL-markierter Sonde um gemeinsame Fangsondenbindestellen kommt. In einem Fall wird dabei die ECL-markierte Indikatorsonde mit 0.4 nmol/l konstant gehalten, die Aequorin-markierte Sonde hingegen im Bereich 0.1 - 10 nmol/l variiert, im anderen Fall wird die Aequorin-Sondenkonzentration festgesetzt auf 1 nmol/l, während die ECL-Sondenkonzentration im Bereich 0.4 - 40 nmol/l variiert wird.

Um direkt vergleichen zu können, wurden die aus diesem Versuch erhaltenen Primärdaten normiert: die ECL-Signale relativ zu 40 nmol/l (= großer Überschuß = 100%), die Aequorin-Signale relativ zu 10 nmol/l (= 100%). Die relativen Änderungen, die sich bei Reaktion der verschieden konzentrierten Fangsondenkonzentrationen mit einer bestimmten Mischung der Indikatorsonden ergaben, wurden gemittelt und anschließend graphisch gegen die variierte Indikatorsondenkonzentration aufgetragen.

### b. Ergebnisse:

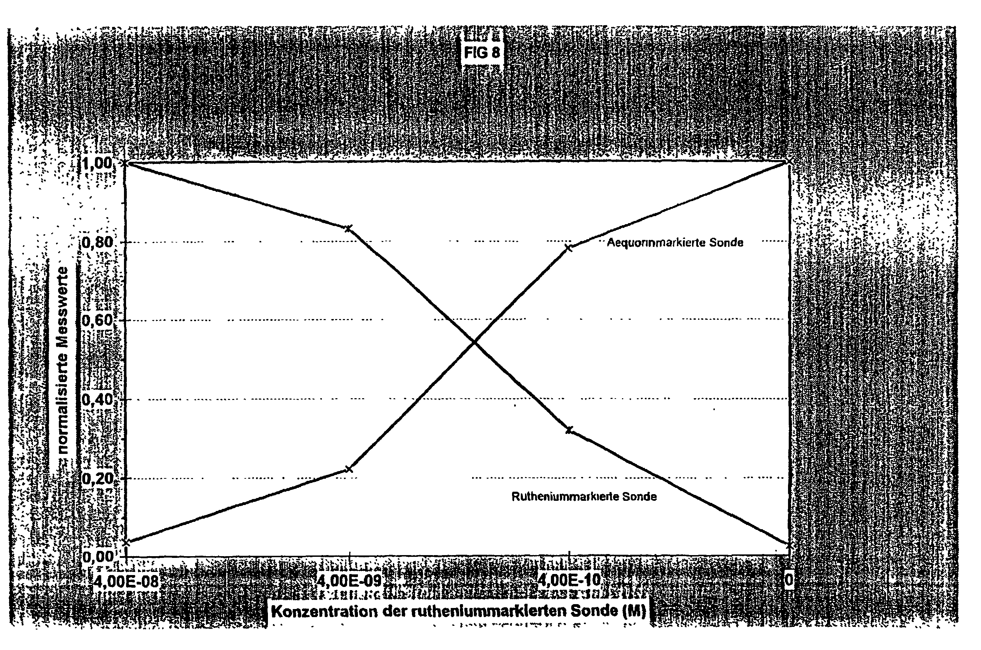

Das Ergebnisse der Messungen ist FIG 7 und 8 zu entnehmen. Bei absolut gleicher Hybridisierungssequenz ist zu erwarten, daß der Schnittpunkt der Kurven genau da liegt, wo die Konzentration der variierten Sonde genau der Konzentration der konstant gehaltenen Sonde entspricht (sollte in der Nähe von 50% liegen).Wird die Aequorin-markierte Sonde auf 1 nmol/l festgesetzt und die Ru²⁺(bipy)₃-markierte Sonde variiert, so erhält man ein Schnittpunkt der Kurven bei ca. 0.8 nmol/l, d.h. 0.8 nmol/l Ru²⁺(bipy)₃-markierte Sonde sind äqui-effizient zu 1.0 nmol/l Aequorin-markierter Sonde, was den zu erwartenden Molmasseneffekt (Aequorin: 22000 Da, Ru²⁺(bipy)₃ < 1000 Da) wiederspiegelt. Umgekehrt erhält man bei Variation der Aequorinmarkierten Sonde einen Schnittpunkt bei ca. 2 nmol/l, d.h. 2 nmol/l sind äqui-effizient zu 0.4 nmol/l Ru²⁺(bipy)₃-markierter Sonde. Diese aus Mittelwerten (s.o.) abgeleiteten Daten zeigen, daß die Hybridisierungsraten mit den beiden Indikatorsonden trotz des erheblichen Molmassenunterschiedes weitgehend ähnlich sind, und die Unterschiede gut der theoretischen Voraussage folgen, womit die prinzipielle Tauglichkeit einer erfindungsgemäßen dual label-Detektion für competitive qPCR auf Basis Co-Amplifikation eines internen Standards unterstrichen wird. Dies wird weiter bestätigt durch die Restaktivität der einen Markierungssonde bei maximalem Überschuß der anderen. Diese beträgt 4% bei konstant 1 nmol/l Aequorin-markierter Sonde und 14% bei konstant 0.4 nmol/l Ru²⁺(bipy)₃-markierter Sonde, was gut den relativen Überschuß der jeweils anderen Sonde von 40:1 im ersten und 25:1 im letzten Fall wiederspiegelt, in der zu erwartenden Orientierung verstärkt durch den Molmasseneffekt (d.h. kinetischer Vorteil der Ru²⁺(bipy)₃-markierten Sonde verstärkt den Effekt des Konzentrationsüberschusses dieser Sonde über die Aequorin-markierte Sonde im ersten Fall, bzw. vermindert den Effekt des Konzentrationsüberschusses der Aequorin-markierten Sonde im letzteren Fall).

### Beispiel 4

### Quantitative Bestimmung einer Nukleinsäure

### Probenvorbereitung und PCR-Amplikation

Eine Rohprobe wird erforderlichenfalls so vorbereitet, daß die Nukleinsäure in zugänglicher Form vorliegen (z. B. Lyse von Zellen etc.). Die Nukleinsäuren können erforderlichenfalls gemäß EP-B-0 201 184 amplifiziert werden (Polymerasekettenreaktion). Gemäß EP 0 420 260 wird Biotin mittels markierter Primer eingebaut.

### Denaturierung

10 µl dieser Reaktionsmischung wurden mit 40 µl Denaturierungslösung (Zusammensetzung
0,05 M Natriumhydroxid, 0,15 M Natriumchlorid) denaturiert.

### Hybridisierung

Anschließend werden 2 Sorten von Sonden zugegeben, von denen eine Rutheniummarkierung (bevorzugt die analytspezifische Sonde) und die andere eine Aequorinmarkierung enthält (bevorzugt die standardspezifische Sonde). Die Sonden sind in einer Hybridisierungslösung mit der Zusammensetzung Phosphatpuffer, pH 6,5, Natriumchlorid und Rinderalbumin gelöst. Von dieser Hybridisierungslösung werden 200 µl zu der vorher erzeugten Reaktionsmischung zugegeben. Das Reaktionsgemisch bei 37 °C fiir 20 Minuten inkubiert.

### Bindung an magnetische Beads

Zu der erhaltenen Reaktionslösung werden 50 µl Lösung von magnetischen Streptavidinbeads (aus Elecsys® TSH-Immunoassay, Boehringer Mannheim GmbH, Best.-Nr. 1731459) (720 µg/ml) zugegeben. Das Reaktionsgemisch wird 20 Minuten bei 37 °C inkubiert.

### Messung

150 µl der erhaltenen Reaktionslösung werden in dem in Figur 1 beschriebenen Gerät in die Meßzelle aufgenommen. Wie in Beispiel 1 beschrieben wird zunächst das Elektrochemilumineszenzsignal und dann das Aequorinsignal gemessen.

### Bezugszeichenliste

- (1): Konditionierlösung fiir die Meßzelle
- (2): Reinigungslösung Für die Meßzelle
- (3): Calciumchloridhaltige Triggerlösung
- (4): Inkubator
- (5): Primärprobenrotor
- (6): beweglicher Pipettor
- (7): Photomultiplier
- (8): beweglicher Magnet
- (9): Referenzelektrode
- (10): Arbeitselektrode
- (11): Gegenelektrode
- (12): Pumpe
- (13): Meßzelle
- (14): Umschaltventil fiir Kolbenpumpe

## Patentansprüche

1. Verfahren zur Bestimmung eines Analyten in einer Probe durch
- Inkubation der Probe mit mindestens zwei Sonden, von denen mindestens eine für den zu bestimmenden Analyten spezifisch ist, wobei die mindestens zwei Sonden unterschiedliche Markierungsgruppen tragen,
- unabhängige Erzeugung jeweils eines elektromagnetischen Signals fiir jede unterschiedliche Markierungsgruppe, dadurch gkennzeichnet, daß ein Signal elektrisch und ein anderes chemisch erzeugt wird
- Auswertung der erzeugten Signale als Zeichen für die Anwesenheit oder Menge des Analyten.

2. Verfahren gemäß Anspruch 1, **dadurch gekennzeichnet, daß** die zweite und gegebenenfalls weitere Sonden spezifisch ist für einen zweiten oder gegebenenfalls weitere Inhaltsstoffe der Probe.

3. Verfahren gemäß Anspruch 2, **dadurch gekennzeichnet, daß** der zweite Inhaltsstoff ein Standardanalyt ist.

4. Verfahren gemäß Anspruch 1 oder 3, **dadurch gekennzeichnet, daß** vor Erzeugung des Signals nicht an den Analyten gebundene analytspezifische Sonde von der analytgebundenen Sonde abgetrennt wird:

5. Verfahren gemäß Anspruch 1 oder 3, **dadurch gekennzeichnet, daß** die unterschiedlichen Markierungsgruppen ausgewählt sind aus Gruppen, die selbst kein für eine Bestimmung ausreichendes elektromagnetisches Signal liefern können, und die Markierungsgruppen nach erfolgter Inkubation mit Reagenzien zur Reaktion gebracht werden, die die Markierungsgruppen in Detektionsgruppen umwandeln, welche jeweils ein elektromagnetisches Signal durch chemische und ein Signal durch elektrische Anregung liefern können.

6. Verfahren gemäß Anspruch 5, **dadurch gekennzeichnet, daß** die unterschiedlichen Markierungsgruppen ausgewählt sind aus der Gruppe der Haptene und/oder Vitamine

7. Verfahren gemäß Anspruch 1 oder 3, **dadurch gekennzeichnet, daß** es sich bei den Markierungsgruppen um Detektionsgruppen handelt, die selbst ein elektromagnetisches Signal liefern können.

8. Verfahren gemäß Anspruch 1, **dadurch gekennzeichnet, daß** die Markierungsgruppen unterschiedlichen lumineszenzfähigen Substanzklassen angehören.

9. Verfahren gemäß Anspruch 8, **dadurch gekennzeichnet, daß** eine der Detektionsgruppen, die selbst ein elektromagnetisches Signal erzeugen, ein durch Ionen aktivierbares Photoprotein und eine andere Detektionsgruppe einen zur Elektrochemilumineszenz anregbaren Metallkomplex enthält.

10. Verfahren gemäß Anspruch 1, 3, 5, 6 oder 7, **dadurch gekennzeichnet, daß** die Signale für die unterschiedlichen Markierungsgruppen zeitlich nacheinander erzeugt werden.

11. Verfahren gemäß Anspruch 1, **dadurch gekennzeichnet, daß** ein erstes Signal ein Elektrochemilumineszenzsignal ist und in Anwesenheit eines Photoproteins erzeugt wird.

12. Reagenzkit zur Bestimmung eines Analyten enthaltend in einem oder getrennten Behältern zwei oder mehr Sonden, von denen mindestens eine für den zu bestimmenden Analyten spezifisch ist, wobei von den mindestens zwei Sonden eine elektrisch zur Lumineszens und eine chemisch zur Lumineszens angeregt werden kann.

13. Kit gemäß Anspruch 12 ferner enthaltend einen Behälter mit einem Standardanalyten.

14. Verwendung zweier Sonden zum quantitativen Nachweis von Analyten in einer Probe, wobei eine ein durch elektrische Anregung erzeugtes und die andere ein durch chemische Anregung erzeugtes Lumineszenzsignale liefern kann.

15. Verfahren zum Nachweis einer Analytnukleinsäure in einer Probe enthaltend
a) Amplifikation zumindest einer Teilsequenz der Analytnukleinsäure sowie zumindest einer Teilsequenz einer definierten Menge einer Standardnukleinsäure in der Probe,
b) Zugabe einer analytnukleinsäurespezifischen Sonde und einer standardnukleinsäurespezifischen Sonde, die jeweils eine elektrisch anregbare und eine chemisch anregbare Markierungsgruppen tragen zu der Probe mit den amplifizierten Nukleinsäuren,
c) Entfernung des Gemisches aus b) aus der Messzelle unter Verbleib der Nukleinsäuren und Sonden in der Messzelle,
d) Überführung der Nukleinsäure in dem Gemisch aus b) in eine Messzelle,
e) Anregung der ersten Markierungsgruppe zur Signalbildung,
f) Messung des durch Anregung in d) gebildeten Signals,
g) Anregung der zweiten Markierungsgruppe zur Signalbildung,
h) Messung des durch Anregung in g) gebildeten Signals und
i) Vergleich der gemessenen Signale zum Nachweis der Analytnukleinsäure.

16. Verfahren zur Bestimmung wenigstens zweier Analyten in einer Probe durch
- Inkubation der Probe mit mindestens zwei Sonden, von denen jeweils eine für die zu bestimmenden Analyten spezifisch ist und wobei diese Sonden unterschiedliche Markierungsgruppen tragen,
- unabhängige Erzeugung jeweils eines elektromagnetischen Signals für jede unterschiedliche Markierungsgruppe, **dadurch gekennzeichnet, daß** ein Signal elektrisch und ein anderes chemisch erzeugt wird
- Auswertung der erzeugten Signale als Zeichen für die Anwesenheit oder die Menge der Analyten.

## Claims

1. Method for the determination of an analyte in a sample by
- incubating the sample with at least two probes of which at least one is specific for the analyte to be determined and the at least two probes carry different labelling groups,
- independently generating an electromagnetic signal for each different labelling group, **characterized in that** one signal is generated electrically and the other is generated chemically
- evaluating the resulting signals as an indication for the presence or amount of the analyte.

2. Method as claimed in claim 1, **characterized in that** the second and optionally further probes are specific for a second component or optionally other components of the sample.

3. Method as claimed in claim 2, **characterized in that** the second component is a standard analyte.

4. Method as claimed in claim 1 or 3, **characterized in that** prior to generating the signal, analyte-specific probe that is not bound to the analyte is separated from the analyte-bound probe.

5. Method as claimed in claim 1 or 3, **characterized in that** the different labelling groups are selected from groups which cannot by themselves generate an adequate electromagnetic signal for a determination and, after incubation, the labelling groups are reacted with reagents which convert the labelling groups into detection groups which can each generate an electromagnetic signal by chemical excitation and a signal by electrical excitation.

6. Method as claimed in claim 5, **characterized in that** the different labelling groups are selected from the group of haptens and/or vitamins.

7. Method as claimed in claim 1 or 3, **characterized in that** the labelling groups are detection groups which themselves can generate an electromagnetic signal.

8. Method as claimed in claim 1, **characterized in that** the labelling groups belong to different substance classes that are capable of luminescence.

9. Method as claimed in claim 8, **characterized in that** one of the detection groups which itself generates an electromagnetic signal contains a photoprotein that can be activated by ions and another detection group contains a metal complex that can be excited to electrochemiluminesce.

10. Method as claimed in claim 1, 3, 5, 6 or 7, **characterized in that** the signals for the different labelling groups are generated successively.

11. Method as claimed in claim 1, **characterized in that** a first signal is an electrochemiluminescence signal and is generated in the presence of a photoprotein.

12. Reagent kit for the determination of an analyte containing in one or separate containers two or more probes of which at least one is specific for the analyte to be determined wherein one of the at least two probes can be excited electrically to luminesce and one can be excited chemically to luminesce.

13. Kit as claimed in claim 12 additionally containing a container containing a standard analyte.

14. Use of two probes for the quantitative detection of analytes in a sample, wherein one can generate luminescence signals by electrical excitation and the other can generate luminescence signals by chemical excitation.

15. Method for the detection of an analyte nucleic acid in a sample comprising
a) amplifying at least a partial sequence of the analyte nucleic acid and at least a partial sequence of a defined amount of a standard nucleic acid in the sample,
b) adding an analyte nucleic acid specific probe and a standard nucleic acid specific probe which each carry an electrically excitable and a chemically excitable labelling group to the sample containing the amplified nucleic acids.
c) removing the mixture from b) from the measuring cell while the nucleic acids and probes remain in the measuring cell,
d) transferring the nucleic acid in the mixture from b) to a measuring cell,
e) exciting the first labelling group to generate a signal,
f) measuring the signal generated by the excitation in d),
g) exciting the second labelling group to generate a signal,
h) measuring the signal generated by the excitation in g) and
i) comparing the measured signals to detect the analyte nucleic acid.

16. Method for the determination of at least two analytes in a sample by
- incubating the sample with at least two probes of which at least one is specific for the analyte to be determined and the at least two probes carry different labelling groups,
- independently generating an electromagnetic signal for each different labelling group, **characterized in that** one signal is generated electrically and the other is generated chemically
- evaluating the resulting signals as an indication for the presence or amount of the analyte.

## Revendications

1. Procédé de détermination d'un analyte dans un échantillon par
- incubation de l'échantillon avec au moins deux sondes, parmi lesquelles au moins une est spécifique pour l'analyte à déterminer, lesdites au moins deux sondes portant des groupes de marquage différents,
- production indépendante d'un signal électromagnétique à chaque fois pour chaque groupe de marquage différent, **caractérisé en ce qu'**un signal est produit électriquement et un autre est produit chimiquement
- évaluation des signaux produits en tant qu'indice de la présence ou de la quantité de l'analyte.

2. Procédé selon la revendication 1, **caractérisé en ce que** la deuxième et éventuellement d'autres sondes sont spécifiques à un deuxième ingrédient ou éventuellement à d'autres ingrédients de l'échantillon.

3. Procédé selon la revendication 2, **caractérisé en ce que** le deuxième ingrédient est un analyte étalon.

4. Procédé selon la revendication 1 ou 3, **caractérisé en ce que**, avant la production du signal, une sonde spécifique à l'analyte, non liée à l'analyte, est séparée de la sonde liée à l'analyte.

5. Procédé selon la revendication 1 ou 3, **caractérisé en ce que**, les différents groupes de marquage sont choisis parmi des groupes qui ne peuvent délivrer eux-mêmes un signal électromagnétique suffisant pour une détermination, et les groupes de marquage après une incubation réussie sont amenés à réagir avec des réactifs qui transforment les groupes de marquage en groupes de détection, lesquels peuvent délivrer respectivement un signal électromagnétique au moyen d'une excitation chimique et un signal au moyen d'une excitation électrique.

6. Procédé selon la revendication 5, **caractérisé en ce que** les groupes de marquage différents sont choisis dans le groupe des haptènes et/ou des vitamines.

7. Procédé selon la revendication 1 ou 3, **caractérisé en ce que**, quant aux groupes de marquage, il s'agit de groupes de détection qui peuvent délivrer eux-mêmes un signal électromagnétique.

8. Procédé selon la revendication 1, **caractérisé en ce que** les groupes de marquage appartiennent à différentes classes de substances capables de luminescence.

9. Procédé selon la revendication 8, **caractérisé en ce que** l'un des groupes de détection qui produisent eux-mêmes un signal électromagnétique, contient une photoprotéine pouvant être activée par des ions et l'autre groupe de détection contient un complexe métallique pouvant être excité pour obtenir la luminescence électrochimique.

10. Procédé selon la revendication 1, 3, 5, 6 ou 7, **caractérisé en ce que**, les signaux sont produits temporellement l'un après l'autre pour les différents marquages.

11. Procédé selon la revendication 1, **caractérisé en ce qu'**un premier signal est un signal de luminescence électrochimique et qu'il est produit en présence d'une photoprotéine.

12. Trousse de réactifs pour déterminer un analyte contenant dans un ou des récipients séparés, deux ou plusieurs sondes, parmi lesquelles au moins une est spécifique à l'analyte à déterminer, une desdites au moins deux sondes pouvant être excitée électriquement pour obtenir la luminescence et une pouvant être excitée chimiquement pour obtenir la luminescence.

13. Trousse selon la revendication 12, contenant de plus un récipient avec un analyte étalon.

14. Utilisation de deux sondes pour la mise en évidence quantitative d'analytes dans un échantillon, l'une pouvant délivrer un signal de luminescence produit par excitation électrique et l'autre pouvant délivrer un signal de luminescence produit par une excitation chimique.

15. Procédé pour la mise en évidence d'un acide nucléique d'analyte dans un échantillon comprenant
a) une amplification d'au moins une séquence partielle de l'acide nucléique de l'analyte et d'au moins une séquence partielle d'une quantité définie d'un acide nucléique étalon dans l'échantillon,
b) l'addition d'une sonde spécifique à l'acide nucléique de l'analyte et d'une sonde spécifique à l'acide nucléique étalon, qui porte respectivement un groupe de marquage pouvant être excité électriquement et un groupe de marquage pouvant être excité chimiquement, à la sonde avec les acides nucléiques amplifiés,
c) l'élimination du mélange issu de b) de la cellule de mesure en conservant les acides nucléiques et les sondes dans la cellule de mesure,
d) transfert de l'acide nucléique dans le mélange issu de b) dans une cellule de mesure,
e) excitation du premier groupe de marquage pour former un signal,
f) mesure du signal formé par excitation dans e),
g) excitation du deuxième groupe de marquage pour former un signal,
h) mesure du signal formé par excitation dans g), et
i) comparaison des signaux mesurés pour mettre en évidence l'acide nucléique de l'analyte.

16. Procédé de détermination d'au moins deux analytes dans un échantillon par
- incubation de l'échantillon au moins deux sondes, dont chacune est spécifique à l'analyte à déterminer et où ces sondes portent des groupes de marquage différents,
- production indépendante à chaque fois d'un signal électromagnétique pour chaque groupe de marquage différent, **caractérisé en ce qu'**un signal est produit électriquement et un autre est produit chimiquement,
- évaluation des signaux produits en tant qu'indice de la présence ou de la quantité des analytes.


### Sequenzprotokoll

(1) ALLGEMEINE ANGABEN:
   (i) ANMELDER:
      (A) NAME: Boehringer Mannheim GmbH
      (B) STRASSE: Sandhoferstr. 116
      (C) ORT: Mannheim
      (E) LAND: DE
      (F) POSTLEITZAHL: 68305
      (G) TELEFON: 0621 759 4348
      (H) TELEFAX: 0621 759 4457
   (ii) BEZEICHNUNG DER ERFINDUNG: Bestimmung von Analyten unter Verwendung zweier Markierungen
   (iii) ANZAHL DER SEQUENZEN: 7
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodesoxyribonukleotid"
   (iii) HYPOTHETISCH: NEIN
   (ix) MERKMAL:
      (A) NAME/SCHLÜSSEL: misc_feature
      (B) LAGE:1
      (D) SONSTIGE ANGABEN:/note= "A am 5'-Ende mit Bio-Link I mit Biotin markiert"
   (ix) MERKMAL:
      (A) NAME/SCHLÜSEL: misc_feature
      (B) LAGE:21
      (D) SONSTIGE ANGABEN:/note= "A am 3'-Ende mit AM III mit BPRu markiert"
   (xi) SEQUENZBESCHREIBUNG: SEQ ID NO: 1:
   AGACCACCAA ATGCCCCTAT A 21
(2) ANGABEN ZU SEQ ID NO: 2:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodesoxyribonukleotid"
   (xi) SEQUENZBESCHREIBUNG: SEQ ID NO: 2:
   CAGAGTTCTA TAGTGCTATG 20
(2) ANGABEN ZU SEQ ID NO: 3:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodesoxyribonukleotid"
   (xi) SEQUENZBESCHREIBUNG: SEQ ID NO: 3:
   CATAGCACTA TAGAACTCTG 20
(2) ANGABEN ZU SEQ ID NO: 4:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodesoxyribonukleotid"
   (xi) SEQUENZBESCHREIBUNG: SEQ ID NO: 4:
   GTCTCTCATC GAGACAAAGT G 21
(2) ANGABEN ZU SEQ ID NO: 5:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodesoxyribonukleotid"
   (xi) SEQUENZBESCHREIBUNG: SEQ ID NO: 5:
   CAGAGTTCTA TAGTGCTATG 20
(2) ANGABEN ZU SEQ ID NO: 6:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 6:
   CAGAGTTCTA TAGTGCTATG 20
(2) ANGABEN ZU SEQ ID NO: 7:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodesoxyribonukleotid"
   (xi) SEQUENZBESCHREIBUNG: SEQ ID NO: 7:
   CATAGCACTA TAGAACTCTG 20